# EUROPEAN PATENT APPLICATION

(11) **EP 1 813 879 A2**
(43) Date of publication of application: **01.08.2007**
(21) Application number: 07002136.5
(22) Date of filing: 31.01.2007
(51) Int. Cl.: F24F 3/16, A61L 9/14, B01D 46/44

(54) **Air filtering apparatus and control method thereof**

(30) Priority: 31.01.2006 JP 2006023021
(71) Applicant: SANYO ELECTRIC CO., LTD., Moriguchi-shi Osaka 570-8667 (JP)
(72) Inventor: Dobashi, Mitsuhiro, Kumagaya-shi Saitama 360-0033 (JP)
(74) Representative: Glawe, Delfs, Moll

(57) **Abstract**

In an air filtering apparatus including a gas-liquid contact member (5) supplied with electrolytic water generated by electrodes (32, 33) and indoor air so that the air is brought into contact with the electrolytic water in the gas-liquid contact member (5), the air being blown out to a room, a performance degrading element other than an operating time of the electrodes or the gas-liquid contact member is achieved, a total amount achieved by weighting the operating time with the performance degrading element is calculated, and then the maintenance timing of the electrodes or the gas-liquid contact member is detected on the basis of the calculated total amount. Thereafter, the maintenance timing when the detecting means detects the maintenance timing is informed to a user.

## Description

### BACKGROUND OF THE INVENTION

### 1. Field of the Invention

The present invention relates to an air filtering apparatus that can remove microorganism such as virus, bacteria, etc. floating in the air, and a control method thereof.

### 2. Description of the Related Art

There has been proposed an air sterilizing apparatus in which electrodes are provided, electrolytic water mist is diffused into the air, and the electrolytic water mist is brought into direct contact with microorganisms floating in the air to inactivate the microorganisms such as virus, bacterial, etc. (for example, see JP-A-2002-181358).

In the conventional air sterilizing apparatus described above, the electrodes are more deteriorated as they are used, and scale is attached to the electrodes, so that the performance of the apparatus is lowered. Furthermore, when electrolytic water mist is diffused, the diffusion range of the electrolytic water mist is limited, and it is difficult for the apparatus to effectively work in a large space, for example, a broad room of a kindergarten, an elementary/junior/high school, long-term care insurance facilities, a hospital or the like.

Furthermore, there has been also proposed an air filtering apparatus in which a gas-liquid contact member is provided, electrolytic water is dropped or infiltrated to the gas-liquid contact member, indoor air is blown to the gas-liquid contact member to bring the indoor air in contact with the electrolytic water water, and then the air is blown out into the room. In this case, the gas-liquid contact member is apart which is required to be subj ected to maintenance, and it is required to accurately specify the maintenance timing of this part.

### SUMMARY OF THE INVENTION

Therefore, an obj ect of the present invention is to provide an air filtering apparatus that can inform a maintenance timing for electrodes or a gas-liquid contact member, and a control method for the air filtering apparatus.

In order to attain the above obj ect, according to an aspect of the presents invention, an air filtering apparatus for filtering microorganisms such as virus, bacteria, etc. is characterized by comprising: air filtering means (12) for supplying a gas-liquid contact member (5) with electrolytic water that is generated through electrolysis of water by at least one pair of electrodes (32, 33), blowing air to the gas-liquid contact member (5) so that the air is brought into contact with the electrolytic water in the gas-liquid contact member (5), and blowing out the air to a room; detecting means (30) for achieving a performance degrading element other than an operating time of the electrodes or the gas-liquid contact member, calculating a total amount achieved by weighting the operating time with the performance degrading element and detecting the maintenance timing of the electrodes or the gas-liquid contact member on the basis of the calculated total amount; and informing means (50, 51) for informing the maintenance timing when the detecting means detects the maintenance timing.

In the above air filtering apparatus, the detecting means (30) may set a time coefficient corresponding to the performance degradation degree based on the performance degrading element on the basis of the performance degrading element, and calculate as the total amount an accumulation value of the multiplication value of the time coefficient and the operating time with respect to the time.

In the above air filtering apparatus, the performance degrading element of the electrodes may be a power value supplied to the electrodes, and the maintenance timing of the electrodes may be detected on the basis of the total amount achieved by weighting the operating time with the power value.

In the above air filtering apparatus, the performance degrading element of the gas-liquid contact member may be an air blowing amount to the gas-liquid contact member, and the maintenance timing of the gas-liquid contact member may be detected on the basis of the total amount achieved by weighting the operating time with the air blowing amount.

According to another aspect of the present invention, a method of controlling an filtering apparatus for supplying a gas-liquid contact member (5) with electrolytic water that is generated through electrolysis of water by at least one pair of electrodes (32, 33), blowing air to the gas-liquid contact member (5) so that the air is brought into contact with the electrolytic water in the gas-liquid contact member (5), and blowing out the air to a room, is characterized by comprising the steps of: achieving a performance degrading element other than an operating time of the electrodes or the gas-liquid contact member; calculating a total amount achieved by weighting the operating time with the performance degrading element; detecting the maintenance timing of the electrodes or the gas-liquid contact member on the basis of the calculated total amount; and informing the maintenance timing when the detecting means detects the maintenance timing.

According to the present invention, the maintenance timing of the electrodes or the gas-liquid contact member is detected on the basis of the total amount achieved by weighting the operating time with the performance degrading element, and thus the maintenance timing suitable for the electrodes and/or the gas-liquid contact member can be informed to the user.

### BRIEF DESCRIPTION OF THE DRAWINGS

Fig. 1 is a perspective view showing an embodiment of the present invention;
Fig. 2 is a perspective view showing the internal construction;
Fig. 3 is a longitudinally-sectional view of a housing;
Fig. 4 is a front view showing a gas-liquid contact member;
Figs. 5A and 5B are systematic diagrams showing the construction of means for dropping electrolytic water to the gas-liquid contact member, wherein Fig. 5A is a side view and Fig. 5B shows the construction of an electrolytic bath;
Fig. 6 is a block diagram showing the construction of a controller;
Fig. 7 is a flowchart showing the detection processing of a maintenance timing of electrodes; and
Fig. 8 is a flowchart showing the detection processing of the maintenance timing of the gas-liquid contact member.

### DETAILED DESCRIPTION OF THE PREFERRED EMBODIMENT

A preferred embodiment according to the present invention will be described hereunder with reference to the accompanying drawings.

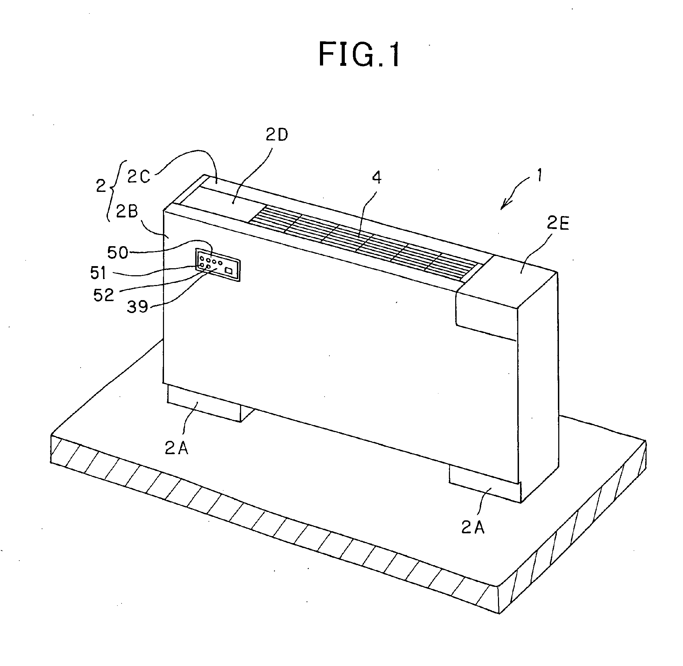

A floor-mount type air filtering apparatus 1 is shown in Fig. 1. The floor-mount type air filtering apparatus 1 has a box type housing 2. The housing 2 contains leg pieces 2A, a front panel 2B and a top panel 2C. An operating lid 2D and an opening/closing lid 2E are laterally arranged at both the sides of the top panel 2C. Furthermore, an informing panel 39 having plural LEDs 50 for informing various kinds of information is disposed at the upper portion of the left side of the front panel 2B. In the following description, "filtering" is broadly defined so as to cover sterilization, removal, inactivation, etc. of microorganisms such as virus, bacteria, fungus, etc.

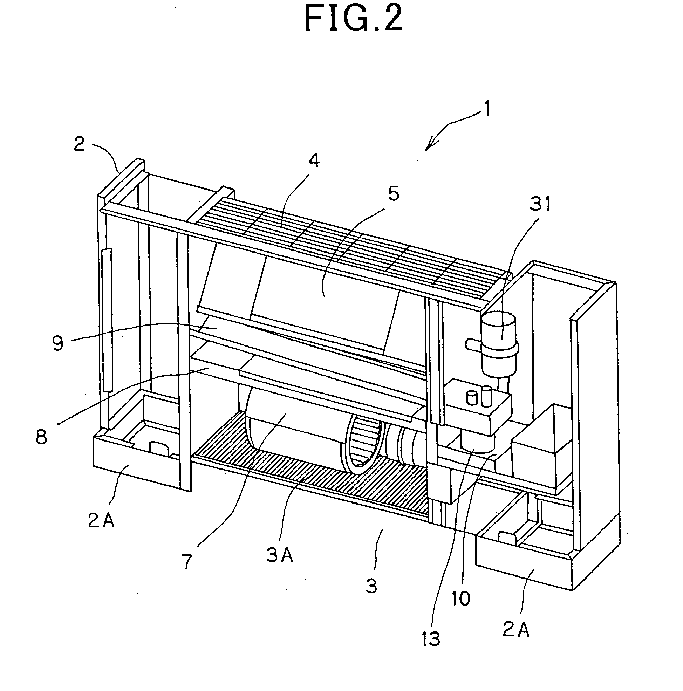

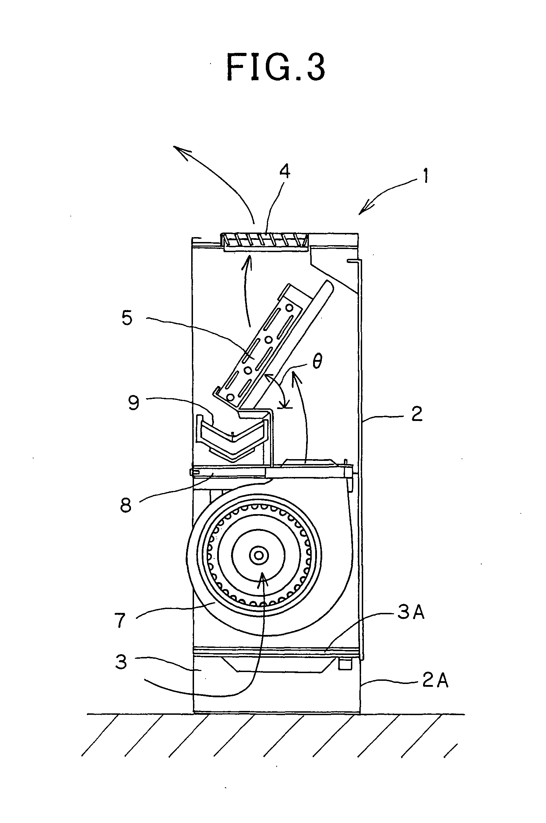

As shown in Fig. 2, an air rectangular suction port 3 is disposed at the lower portion of the housing 2, and a pre-filter 3A is disposed above the air suction port 3. An air blowing fan 7 is supported above the pre-filter 3A through a support plate 8 in the housing 2. A gas-liquid contact member 5 having high water retentivity is disposed above the support plate 8 while tilted as shown in Fig. 3. A water receiving tray 9 is disposed between the gas-liquid contact member 5 and the air blowing fan 7, and an laterally elongated air blowing port 4 is disposed above the gas-liquid contact member 5.

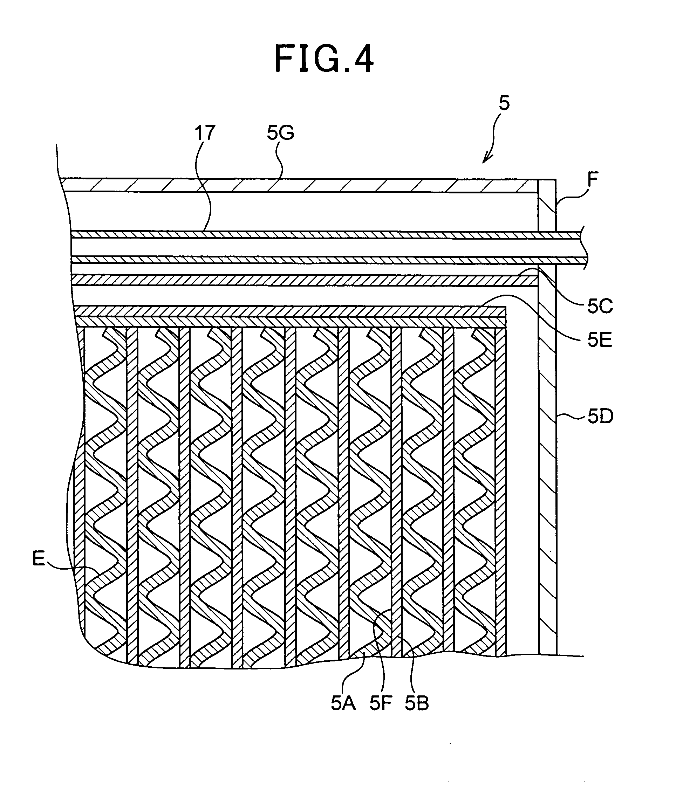

As shown in Fig. 4, the gas-liquid contact member 5 is constructed by an element portion E, and a frame portion F for supporting the element portion E.

The element portion E is constructed by laminating corrugated plate type members 5A and flat plate type members 5B. Therefore, substantially triangular plural openings 5F are formed between each corrugated plate type member 5A and each flat plate type member 5B.

The frame portion F has a pair of frame members 5D constituting both the end frames of the gas-liquid contact member 5, an electrolytic water supply pipe 17 that penetrates through one frame member 5D and is fixed to the other frame member 5D at the tip thereof, and a cover 5G that is fixed between the upper end portions of the pair of frame members 5D so as to cover the electrolytic water supply pipe 17 and constitutes the upper frame of the gas-liquid contact member 5, and the cover 5G supports a first distributing sheet 5C disposed below the electrolytic water supply pipe 17. The element portion E is disposed between the pair of the frame members 5D, and plural second distributing sheets 5E are disposed on the upper surface of the element portion E.

The corrugated plate type members 5A, the flat plate type members 5B, the first distributing sheet 5C and the second distributing sheet 5E are formed of fiber raw materials having liquid permeability. Electrolytic water discharged from many spray holes (not shown) formed in the electrolytic water supply pipe 17 is dropped to the first distributing sheet 5C, whereby the electrolytic water is diffused to the whole of the first distributing sheet 5C, and drops onto the whole upper surface of the element portion E. the dropped electrolytic water infiltrates through the second distributing sheet 5E, and diffuses into the whole of the second distributing sheet 5E to supply the electrolytic water to the whole upper portion of the element portion E, whereby the electrolytic water infiltrates to the corrugated plate type members 5A and the flat type members 5B of the element portion E in every corner.

In this case, raw material having little deterioration to electrolytic water, for example, polyolefin resin (polyethylene resin, polypropylene resin or the like), PET (polyethylene terephthalate) resin, vinyl chloride resin, fluorinated resin (PTFE, PFA, ETFE or the like), ceramic material or the like is used as the raw materials of the corrugated plate type members 5A, the flat plate type members 5B, the first distributing sheet 5C and the second distributing sheet 5E. PET resin is used in this embodiment. Electrolytic water has a fungicidal property, and thus it is unnecessary to coat fungicide on the gas-liquid contact member 5.

As shown in Fig. 3, the inclination (tilt) angle θ of the gas-liquid contact member 5 is preferably set to 30° or more. I f the inclination angle is less than 30°, the dropped electrolytic water does not flow along the slope of the gas-liquid contact member 5, but falls downwardly. Furthermore, if the inclination angle approaches to 90°, the flowing direction of air passing through the gas-liquid contact member 5 is substantially horizontal and thus it is difficult to blow the air upwardly. When the blow-out direction of the air is approached to the horizontal direction, it is impossible to blow the air far away. In this case, the air filtering apparatus is not suitable to sterilize air in a large space. It is preferable that the inclination angle θ satisfies the condition: 80° > θ > 30°, and it is more preferable that the inclination angle θ satisfies the condition: 75° > θ > 55°. In this embodiment, the inclination angle is set to about 57°.

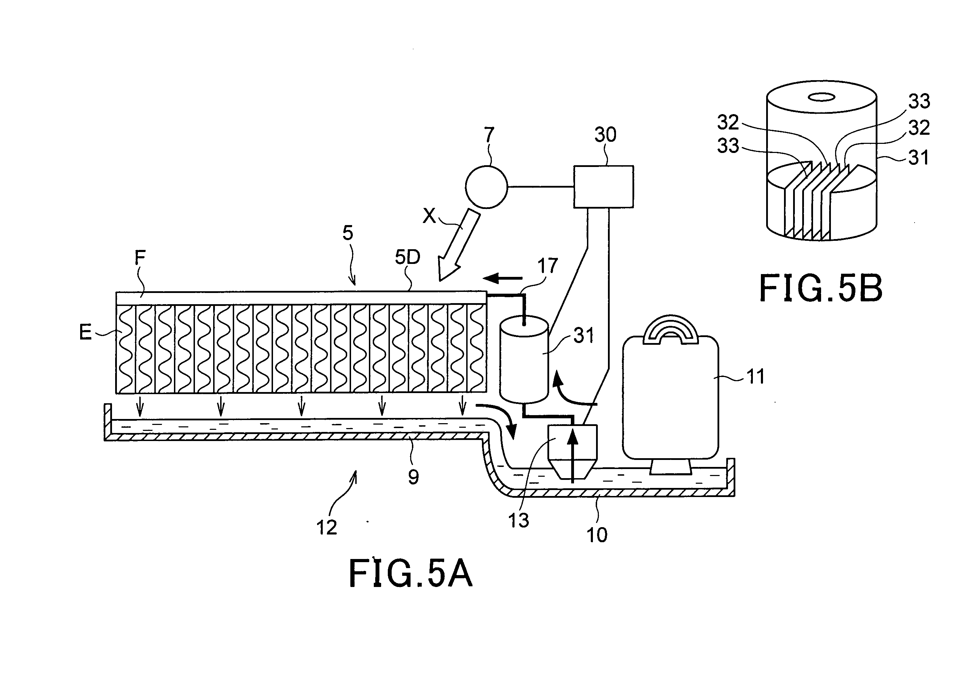

Figs. 5Aand 5B show an electrolyticwater supply mechanism for dropping electrolytic water to the gas-liquid contact member 5.

A water receiving tray 9 is disposed below the gas-liquid contact member 5, and a water supplying support tank support tray 10 intercommunicates with the water receiving tray 9. A water supply tank 11 for supplying water containing chlorine ions (the water may be tap water or the like) and a circulating pump 13 are disposed on the support tray 10. An electrolytic bath 31 is connected to the circulating pump 13 and an electrolytic water supply pipe 17 is connected to the electrolytic bath 31.

As shown in Fig. 5A, the electrolytic bath 31 is disposed above the water receiving tray 9 and the water supply tank support tray 10. When the operation of the circulating pump 13 is started, water pumped up from the water supply tank support tray 10 is stocked in the electrolytic bath 31, and when the operation of the circulating pump 13 is stopped, the water in the electrolytic bath 31 naturally drops to the water supply tank support tray 10 by gravitation, so that the electrolytic bath 31 is empty.

As shown in Fig. 5, at least one pair of electrodes 32 and 33 which serve as an anode and a cathode are disposed (alternately disposed in the case of use of two or more pairs) in the electrolytic bath 31, and when current is supplied to the electrodes 32 and 33, water (tap water) flowing in the electrolytic bath 31 is electrolyzed to generate active oxygen specifies. Here, the active oxygen species means oxygen molecules having higher oxidizing activity than normal oxygen and also related substance thereof, and contain not only so-called narrowly-defined active oxygen such as superoxide anion, singlet oxygen, hydroxyl radical and hydrogen peroxide, but also so-called broadly-defined active oxygensuch asozone,hypohalous acid, etc. The electrolytic bath 31 is disposed in proximity to the gas-liquid contact member 5, and it is designed so as to immediately supply the gas-liquid contact member 5 with active oxygen specifies generated by electrolyzing water (tap water) .

The electrodes 32, 33 are electrode plates each of which comprises a base of Ti (titan) and a coated layer of Ir (iridium), Pt (platinum) . The current value applied to the electrodes 32, 33 is set so as to be equal to several MA (milliampere) /cm² (square centimeter) to several tens mA /cm², and a predetermined concentration of free residual chlorine (for example, 1 mg/l liter) is generated, for example.

When current is supplied to tap water by the electrodes 32, 33, the following reaction occurs at the cathode:

4H⁺ + 4e⁻ + (4OH⁻) → 2H₂ + (4OH⁻)

Furthermore, the following reaction occurs at the anode:

2H₂O → 4H⁺ + O₂ + 4e⁻

At the same time, chlorine ions contained water (chlorine ions are added in tap water in advance) reacts as follows:

2Cl⁻ → Cl₂ + 2e⁻

Furthermore, Cl₂ thus generated reacts with water as follows:

Cl₂ + H₂O → HClO + HCl

In this construction, by supplying current to the electrodes 32, 33, HClO (hypochlorous acid) having strong sterilizingpower is generated. Therefore, air ispassed through the gas-liquid contact member 5 supplied with this hypochlorous acid, thereby preventing breeding of various bacteria in the gas-liquid contact member 5. Accordingly, virus, bacteria, etc. floating in the air passing through the gas-liquid contact member 5 can be inactivated. Furthermore, when odor components pass through the gas-liquid contact member 5, the odor components also react with hypochlorous acid in the electrolytic water, and they are ionized and dissolved, whereby the odor components can be removed from air and the air is deodorized. That is, the electrolytic bath 31, the gas-liquid contact member 5, the air blowing fan 7 and the circulating pump 13 constitutes the air filtering mechanism (air filtering means) 12.

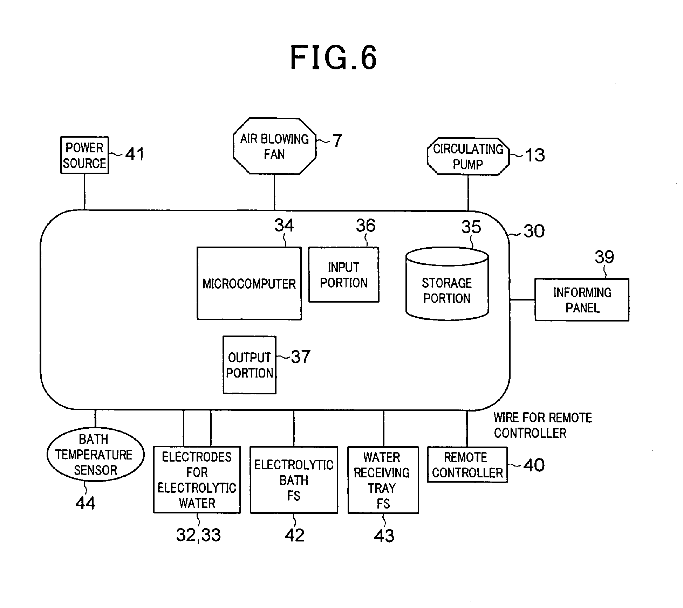

The floor-mount type air filtering apparatus 1 is equipped with a controller 30 for centrically controlling each part of the floor-mount type air filtering apparatus 1 . Fig. 6 is a block diagram showing the controller 30 and the peripheral construction.

The controller 30 is equipped with a microcomputer 34, a storage portion 35, an input portion 36 and an output portion 37, and it inputs various instructions from a user through an operating panel exposed to the outside when an operating lid 2D is opened, and a remote controller 40 for remote operation (hereinafter referred to as "remote controller").

The microcomputer 34 controls the electrolytic bath 31, the air blowing fan 7, the circulating pump 13, etc. on the basis of control programs stored in advance in the storage 35 as a non-volatile memory such as EEPROM or the like. The controller 30 achieves a current value I supplied to the electrodes 32, 33, an electrolytic time (operating time) t t1 corresponding to an electrolysis time, an air blowing speed ν of the air blowing fan 7 and an air blowing time t2 when the electrolytic bath 31 carries out electrolysis, and stores these information into the storage portion 35. In this embodiment, two air blowing speeds such strong wind and weak wind are provided as the air blowing speed v.

The input portion 36 is an interface for receiving detection signals from the electrodes 32, 33, the bath temperature sensor 44, an electrolytic bath float switch 42 and a water receiving tray float switch 43, and the output portion 37 outputs power to the electrodes 32, 33.

The microcomputer 34 issues a water supply request when it receives a no-water detection signal from the water receiving tray float switch 43 through the input portion 36, and turns on a water supply informing lamp (not shown) disposed on the informing panel 39.

The microcomputer 22, the storage portion, the input portion 36 and the output portion 37 are mounted on a board, and accommodated in an electrical component box (not shown).

Next, a power source 41, the bath temperature sensor 44, the electrolytic bath float switch 42, the water receiving tray float switch 43, the electrodes 32, 33 and the informing panel 39 which are connected to the controller 30 will be described.

The power source 41 is a power source for operating the floor-mount type air filtering apparatus 1, and when the power supply switchof the operatingpanel is turned on, power is supplied to the equipment connected to the controller 30.

A temperature sensor disposed in the electrolytic bath 31 is applied as the bath temperature sensor 44, and detects the temperature of the electrolytic water in the electrolytic bath 31.

The electrolytic bath float switch 42 detects whether the water level of electrolytic water in the electrolytic bath 31 is equal to a predetermined permissible water level or more.

The water receiving tray float switch 43 detects whether the water level of electrolytic water in the water receiving tray 9 is equal to a predetermined permissible water level or more.

The electrodes 32, 33 provided to the electrolytic bath 31 is used not only for electrolysis of water such as tap water or the like, but also as a detection electrode for detecting the electric conductivity of the electrolytic water.

As described above, the informing panel 39 serves as informing means for informing various kinds of information such as information indicating that LED 50 is turned on and the apparatus is being operated, etc. At the upper stage of the informing panel 39 (Fig. 1) are disposed a green lamp for informing that the apparatus is under operation, a red lamp for promoting supply of water to the water supply tank 11, a red lamp for informing the maintenance timing of the pre-filter 3A, and a red lamp for promoting water exchange of the water receiving tray 9. Furthermore, at the lower stage of the informing panel 39 are disposed an electrode exchange lamp 51 (Fig. 1) for informing the maintenance timing caused by the deterioration of the electrodes 32, 33, and a gas-liquid contact member exchange lamp 52 (Fig. 1) for informing the maintenance timing caused by the deterioration of the gas-liquid contact member 5.

Next, the operation of the floor-mount type air filtering apparatus 1 during the air filtering operation will be described.

In Fig. 1, when the operating lid 2D is opened, the operating panel (not shown) is exposed to the outside. The operating panel is operated to start the operation of the floor-mount type air filtering apparatus 1. When this operation is started, the circulating pump 13 is driven, and water (tap water or the like) stocked in the water supply tank support tray 10 is supplied to the electrolytic bath 31 as shown in Fig. 5A. In this electrolytic bath 31, current is supplied to the electrodes 32, 33 to electrolyze the water, thereby generating electrolytic water containing active oxygen specifies. The electrolytic water is passed through the water spray holes (not shown) formed in the electrolytic water supply tube 17, and sprayed into the diffusion holes 5C. Then, the electrolytic water infiltrates into the upper edge portion of the gas-liquid contact member 5 from the diffusion holes 5C and then gradually infiltrates to the lower portion.

The electrolytic water dropped from the gas-liquid contact member 5 is received by the water receiving tray 9, flows along a slant face at one side thereof into the water supply tank support tray 10 and is stocked there. In this construction, a water circulating system is used, and thus when the amount of water is reduced due to vaporization or the like, a proper amount of water such as tap water in the water supply tank 11 is supplied to the water supply tank support tray 10. The water supply tank 11 is disposed so as to be freely detached when the open/close lid 2E (see Fig. 1) is opened, so that it can be supplemented with water such as tap water or the like by detaching the water supply tank 11.

As indicated by an arrow X, indoor air is supplied through the air blowing an 7 to the gas-liquid contact member 5 into which the electrolytic water infiltrates. The indoor air comes into contact with the active oxygen specifies infiltrating in the gas-liquid contact member 5, and then blown out to the room again. For example when influenza virus floats in indoor air, the active oxygen species function to break down and vanish (remove) the surface protein (spike) of the virus concerned which is indispensable for infection. When the surface protein of influenza virus is broken down, the influenza virus is not joined to a receptor which is necessary for infection of the virus concerned, so that infection can be prevented. As a result of a verification test, it has been found that when air in which influenza virus floats is passed through the gas-liquid contact member 5 of this embodiment, 99% or more of the virus concerned can be removed.

With respect to the electrodes 32, 33, platinum is gradually wasted (deteriorated) as electrolysis is conducted, and also scale generated through electrolysis adheres to the electrodes, so that the performance of the electrodes is degraded. Furthermore, with respect to the gas-liquid contact member 5, the constituent elements are gradually dissolved by electrolytic water, and also gradually deteriorated by impurities contained in soiled air or electrolytic water.

Therefore, this embodiment is equipped with a function of executing the processing of detecting the maintenance timing of the electrodes 32, 33 and the processing of detecting the maintenance timing of the gas-liquid contact member 5 and informing these maintenance timings to the user.

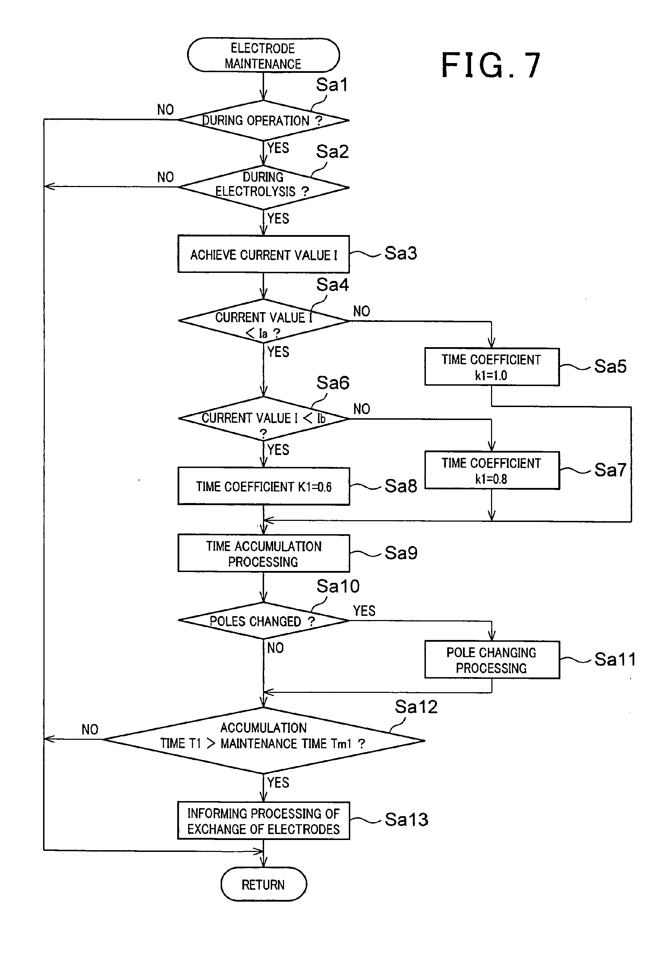

Fig. 7 is a flowchart showing the processing of detecting the maintenance timing of the electrodes 32, 33. This detection processing is repetitively executed when the floor-mount type air filtering apparatus 1 is powered.

First, when the power of the floor-mount type air filtering apparatus 1 is turned on, the controller 30 judges whether the system of the apparatus 1 is under operation or not (step Sa1). When the operation of the system is stopped (step Sa1 : No), the controller 30 temporarily finishes the detection processing of the maintenance timing because the electrodes 32 and 33 are not wasted.

If it is judged that the system is under operation (step Sa1: Yes), the controller 30 judges whether electrolysis is being conducted in the electrolytic bath 31 (step Sa2). If no electrolysis is being conducted (step Sa2: No), the controller 30 temporarily finishes the detection processing of the maintenance timingbecause the electrodes 32 and 33 are not wasted.

If it is judged that the electrolysis is being conducted (step Sa2: Yes), the controller 30 achieves the current value I of power output to the electrodes 32 and 33 of the electrolytic bath 31 (step Sa3), and in order to detect the waste degree of the electrodes 32 and 33, it is judged whether the current value I is less than a preset threshold value Ia (step Sa4). If it is judged that the current value I is not less than the threshold value Ia, the controller 30 sets a time coefficient k1 to the value (1.0) corresponding to the waste degree of the electrodes 32 and 33 when the current value I is not less than the threshold value Ia (step Sa5), and the processing shifts to step Sa9.

On the other hand, if it is judged that the current value I is less than the preset threshold value (step Sa4: Yes), the controller 30 is judged whether the current value I is less than a preset threshold value Ib (step Sa6) . Here, the threshold value Ib is set to be smaller than the threshold value Ia. If it is judged that the current value I is not less than the threshold value Ib (step Sa6: No), the controller 30 sets the time coefficient k1 to the value (0.8) corresponding to the waste degree of the electrodes 32 and 33 when the current value I is not less than the threshold value Ib and also is less than the threshold value Ia (step Sa7), and the processing is shifted to step Sa9.

On the other hand, if the current value I is less than the threshold value Ib (step Sa6: Yes), the controller 30 sets the time coefficient k1 to the value (0.6) corresponding to the waste degree of the electrodes 32 and 33 when the current value I is less than the threshold value Ib (step Sa8) . When the time coefficient k1 corresponding to the waste degree of the electrodes 32 and 33 is set on the basis of the current value I, the controller 30 executes the time integrating processing (step Sa9).

According to the time integrating processing, the controller 30 multiplies the time coefficient k1 by the operating time t1, and accumulates these multiplication values, thereby calculating the accumulated time (total amount) T1 which is weighted by the operating time t and the time coefficient k1.

Subsequently, the controller 30 judges whether a pole unchanging time elapses a preset time (for example, 15 minutes), thereby judging whether the poles of the electrodes 32 and 33 are inverted to each other (step Sa10) . If the pole unchanging time does not elapse the preset time (step Sa10:No), the processing shifts to step Sa12. On the other hand, if the pole unchanging time elapses the preset time (step SalO: Yes), the pole changing processing (step Sa11) is conducted, and then the processing shifts to step Sa12. By carrying out the pole changing processing as described above, the amount of platinum of the electrodes 32 and 33 and the adherence amount of scale can be averaged, and the lifetime of the electrodes 32 and 33 can be lengthened.

In step Sa12, in order to judge whether the electrodes 32 and 33 reach the maintenance timing, the controller 30 judges whether the accumulation time T1 exceeds a preset maintenance time Tm1 of the electrodes 32 and 33 (step Sa12). When it is judged that the accumulation time T1 does not exceed the maintenance time Tm1 (step Sa12: No), the controller 30 temporarily finishes the detection processing of the maintenance timing, and repeats a series of detection processing of the maintenance timing.

On the other hand, if the accumulation time T1 exceeds the maintenance time Tm1 (step Sa12: Yes), the controller 30 executes the informing processing of turning on the electrode exchange lamp (Fig. 1) to promote the exchange of the electrodes 32 and 33 (step Sa13).

Here, when the informing processing of promoting the exchange of the electrodes 32 and 33 is executed, the user exchanges the electrodes 32 and 33. At this time, when the user operates an exchange finishing button switch (not shown) which is provided to the remote controller 40 to inform the completionof the exchange of the electrodes 32 and 33, the controller 30 turns off the electrode exchange lamp 51 and resets the count value of the accumulation time T1.

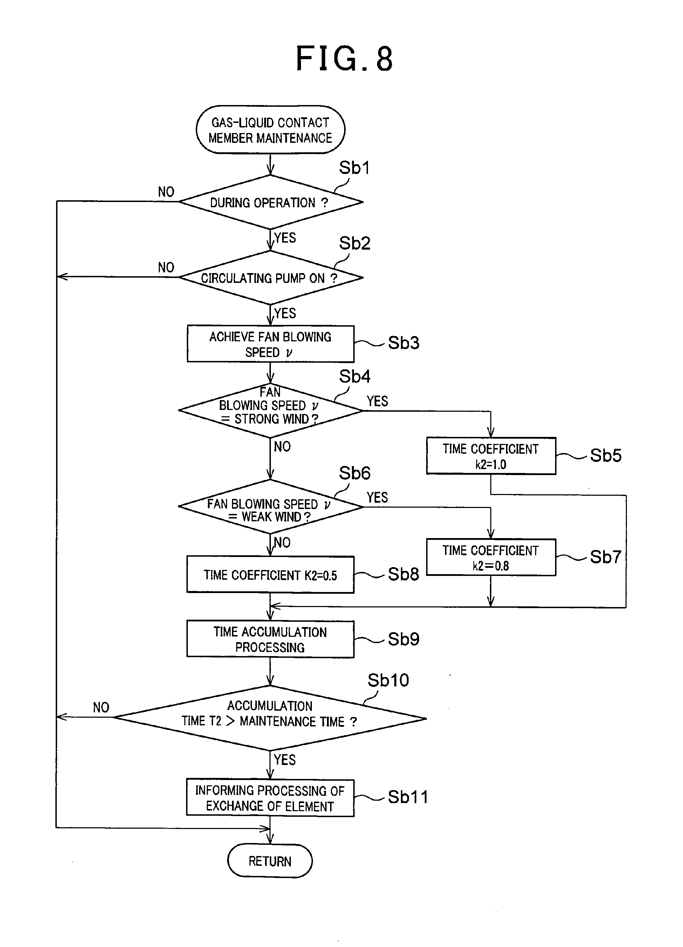

Fig. 8 is a flowchart showing the detection processing of the maintenance timing of the gas-liquid contact member 5. This detection processing is repetitively executed when the floor-mount type air filtering apparatus is powered.

First, when the floor-mount type air filtering apparatus 1 is powered, the controller 30 judges whether the system of the apparatus is under operation (step Sb1). When the operation of the system is stopped (step Sb1: No), the controller 30 temporarily finishes the detection processing of the maintenance timing because the gas-liquid contact member 5 is not deteriorated.

When it is judged that the system is under operation (step Sb1: Yes), the controller 30 judges whether the circulating pump is under operation (step Sb2). When the operation of the circulating pump 13 is stopped (step Sb2: No), the controller 30 temporarily finishes the detection processing of the maintenance timing because the gas-liquid contact member 5 is not deteriorated by electrolytic water.

When it is judged that the circulating pump 13 is under operation (step Sb2: Yes), the controller 30 achieves the air blowing speed ν of the air blowing fan 7 (step Sb3), and in order to detect the deterioration degree of the gas-liquid contact member 5, the controller 30 judges whether the air blowing speed ν corresponds to the strong wind (step Sb4) . When the air blowing speed ν corresponds to the strong wind (step Sb4: Yes), the controller 30 sets the time coefficient k2 to the value (1.0) corresponding to the deterioration degree of the gas-liquid contact member 5 under strong wind (step Sb5), and shifts the processing to step Sb9.

On the other hand, if the air blowing speed ν does not correspond to the strong wind (step Sb4: No), the controller 30 judges whether the air blowing speed ν corresponds to the weak wind (step Sb6) . If it is judged that the air blowing speed ν corresponds to the weak wind (step Sb6: Yes), the controller 30 sets the time coefficient k2 to the value (0.8) corresponding to the deterioration degree of the gas-liquid contact member 5 under the weak wind (step Sb7), and shifts the processing to step Sb9.

On the other hand, when the air blowing speed ν does not correspond to the weak wind (step Sb6: No), that is, when the air blowing fan 7 is not operated, the controller 30 sets the time coefficient k2 to the value (0.5) corresponding to the deterioration degree of the gas-liquid contact member 5 under no wind (step Sb8) . When the time coefficient k2 corresponding to the waste degree (deterioration degree) of the gas-liquid contact member 5 is set on the basis of the air blowing speed ν as described above, the controller 30 executes the time accumulation processing (step Sb9).

According to the time accumulation processing, the controller 30 multiplies the time coefficient k2 by the operating time t2, and accumulates these multiplication values, thereby calculating the accumulation time (total amount) T2 which is weighted by the operating time t2 and the time coefficient k2.

Next, in order to judge whether the gas-liquid contact member 5 reaches the maintenance timing, the controller 30 judges whether the accumulation time T2 exceeds a preset maintenance time Tm2 of the gas-liquid contact member 5 (step Sb10). If it is judged that the accumulation time T2 does not exceed the maintenance time Tm2 (step Sb: No), the controller 30 temporarily finishes the detection processing of the maintenance timing, and repeats the series of detection processing of the maintenance timing.

On the other hand, if the accumulation time T2 exceeds the maintenance time Tm2 (step Sa10: Yes), the controller 30 executes the informing processing of turning on the gas-liquid contact member exchange lamp 52 (Fig. 1) and promoting the exchange of the gas-liquid contact member 5 (step Sb11) .

Here, when the informing processing of promoting the exchange of the gas-liquid contact member 5 is executed, the user exchanges the gas-liquid contact member 5. In this case, when the user operates an exchange end button switch (not shown) that is provided to the remote controller 40 and informs the end of the exchange of the gas-liquid contact member 5, the controller 30 turns off the gas-liquid contact member exchange lamp 52, and resets the count value of the accumulation time T2.

As described above, according to this embodiment, the current value I of power supplied to the electrodes 32 and 33 is achieved as a performance degrading element, the time coefficient k1 corresponding to the performance degradation degree based on the achieved current value I is set, the operating time t1 is weighted by the time coefficient k1, and then the accumulation time T1 is calculated. Therefore, there can be achieved the accumulation time T2 which is substantially coincident with the performance degradation degree of the electrodes 32 and 33 which are more degraded in performance as the supplied power is larger. Accordingly, the maintenance timing of the electrodes 32 and 33 can be properly detected on the basis of the accumulation time T1, and it can be informed to the user.

Furthermore, the air blowing speed ν of air supplied from the air blowing fan 7 to the gas-liquid contact member 5 is achieved as a performance degrading element, the time coefficient k2 corresponding to the performance degradation degree based on the achieved air blowing speed ν is set, the operating time t2 is weighted by the time coefficient k2, and then the accumulation time T2 is calculated. Therefore, there can be achieved the accumulation time T2 which is substantially equal to the performance degradation degree of the gas-liquid contact member 5 which is more degraded in performance as the supplied air blowing amount is larger. Accordingly, the maintenance timing of the gas-liquid contact member 5 can be properly detected on the basis of the accumulation time T2, and informed to the user.

The present invention is not limited to the above-described embodiment. For example, the air filtering apparatus of the present invention may be designed so that ozone (O₃) or hydrogen peroxide (H₂O₂) is generated as the active oxygen species. In this case, when platinum tantalum electrodes are used as the electrodes, active oxygen species can be highly efficiently and stably generated from water in which ion species are rare.

At this time, at the anode, the following reaction occurs:

2H₂O → 4H⁺ + O₂ + 4e⁻

Simultaneously with the above reaction, the following reactions occur, and ozone (O₃) is generated.

3H₂O → O₃ + 6H⁺ + 6e⁻

2H₂O → O₃ + 4H⁺ + 4e⁻

Furthermore, at the cathode, the following reactions occur:

4H⁺ + 4e⁻ + (4OH⁻) → 2H₂ + (4OH⁻)

O₂⁻ + e⁻ + 2H⁺ → H₂O₂

That is, O₂⁻ generated through the electrode reaction and H⁺ in solution are bonded to each other to generate hydrogen peroxide (H₂O₂).

In this construction, ozone (O₃) and hydrogen peroxide (H₂O₂) which have strong sterilizing power are generated by supplying current to the electrodes, and electrolytic water containing ozone (O₃) and hydrogen peroxide (H₂O₂) can be created. The concentration of ozone or hydrogen peroxide in the electrolytic water is adjusted to a value suitable for inactivate target virus or the like and air is passed through the gas-liquid contact member 5 supplied with the electrolytic water having this concentration, whereby target virus, etc. floating in the air can be inactivated. Furthermore, even odor reacts with ozone or hydrogen peroxide in the electrolytic water when passing through the gas-liquid contact member 5, and ionized and dissolved in the electrolytic water, whereby the odor components are removed from the air and thus the air is deodorized.

In the above embodiment, the electrolytic water dropping means to the gas-liquid contact member 5 is described. However, the present invention is not limited to this style. Electrolytic water may be infiltrated into the gas-liquid contact member 5. In this case, electrolytic water may be stocked in the water receiving tray 9 so that the lower edge portion of the gas-liquid contact member 5 is submerged in the electrolytic water, whereby the electrolytic water is sucked up by a so-called capillary phenomenon.

In the above embodiment, the current value I and the air blowing speed ν are achieved as the performance degrading elements of the electrodes 32 and 33 and the gas-liquid contact member 5, however, the present invention is not limited to this embodiment. For example, a power value or voltage value supplied to the electrodes 32 and 33 may be achieved as the performance degrading element of the electrodes 32 and 33, and the rotational number of the air blowing fan 7, the supply amount of electrolytic water by the circulating pump 13, the rotational number of the circulating pump 13 or the like may be used as the performance degrading element of the gas-liquid contact member 5.

Furthermore, in the above embodiment, the maintenance timing is informed by using the electrode exchange lamp 51 and the gas-liquid contact member exchange lamp 52. However, the present invention is not limited to this embodiment, and the maintenance timing may be informed by using not only a display device such as a lamp, but also a sound emitting device such as a buzzer or the like.

## Claims

1. An air filtering apparatus for filtering microorganisms such as virus, bacteria, etc., **characterized by** comprising:
air filtering means (12) for supplying a gas-liquid contact member (5) with electrolytic water that is generated through electrolysis of water by at least one pair of electrodes (32, 33), blowing air to the gas-liquid contact member (5) so that the air is brought into contact with the electrolytic water in the gas-liquid contact member (5), and blowing out the air to a room;
detecting means (30) for achieving a performance degrading element other than an operating time of the electrodes or the gas-liquid contact member, calculating a total amount achieved by weighting the operating time with the performance degrading element and detecting the maintenance timing of the electrodes or the gas-liquid contact member on the basis of the calculated total amount; and
informing means (50, 51) for informing the maintenance timing when the detecting means detects the maintenance timing.

2. The air filtering apparatus according to claim 1, wherein the detecting means (30) sets a time coefficient corresponding to the performance degradation degree based on the performance degrading element on the basis of the performance degrading element, and calculates as the total amount an accumulation value of the multiplication value of the time coefficient and the operating time with respect to the time.

3. The air filtering apparatus according to claim 1 or 2, wherein the performance degrading element of the electrodes is a power value supplied to the electrodes, and the maintenance timing of the electrodes is detected on the basis of the total amount achieved by weighting the operating time with the power value.

4. The air filtering apparatus according to any one of claims 1 to 3, wherein the performance degrading element of the gas-liquid contact member is an air blowing amount to the gas-liquid contact member, and the maintenance timing of the gas-liquid contact member is detected on the basis of the total amount achieved by weighting the operating time with the air blowing amount.

5. A method of controlling an filtering apparatus for supplying a gas-liquid contact member (5) with electrolytic water that is generated through electrolysis of water by at least one pair of electrodes (32, 33), blowing air to the gas-liquid contact member (5) so that the air is brought into contact with the electrolytic water in the gas-liquid contact member (5), and blowing out the air to a room, **characterized by** comprising the steps of:
achieving a performance degrading element other than an operating time of the electrodes or the gas-liquid contact member;
calculating a total amount achieved by weighting the operating time with the performance degrading element;
detecting the maintenance timing of the electrodes or the gas-liquid contact member on the basis of the calculated total amount; and
informing the maintenance timing when the detecting means detects the maintenance timing.
